# FASCICULE DE BREVET EUROPEEN

(11) **EP 3 361 993 B1**
(45) Date de publication et mention de la délivrance du brevet: **31.07.2019**
(21) Numéro de dépôt: 16793959.4
(22) Date de dépôt: 11.10.2016
(51) Int. Cl.: A61F 2/38

(54) **IMPLANT CONDYLIEN POUR PROTHESE DE GENOU**
KONDYLENIMPLANTAT FÜR EINE KNIEPROTHESE
CONDYLAR IMPLANT FOR A KNEE PROSTHESIS

(30) Priorité: 12.10.2015 FR 1559658
(43) Date de publication de la demande: 22.08.2018
(73) Titulaire: One Ortho, 69230 Saint-Genis-Laval (FR)
(72) Inventeur: GUITON, Thierry, 76100 Rouen (FR); ALEPEE, Christophe, 69003 Lyon (FR); PFEFFER, Frédéric, 88390 Les Forges (FR); ESTOUR, Gilles, 73000 Chambery (FR); GANDON, Didier, 51100 Reims (FR); GALY, Charles, 31800 Latoue (FR)
(74) Mandataire: Cabinet Laurent & Charras
(86) Numéro de dépôt international: PCT/FR2016/052624
(87) Numéro de publication internationale: WO 2017/064410

(56) Documents cités:
- WO-A1-2004/016204
- US-A- 4 340 978
- US-A- 6 013 103
- US-A- 6 152 960

## Description

### DOMAINE TECHNIQUE

La présente invention se rapporte au secteur technique de la chirurgie orthopédique, et concerne plus particulièrement un implant condylien pour prothèse de genou, de préférence uni-compartimentale, par exemple pour une arthroplastie partielle du genou pour les patients présentant une usure localisée au niveau du condyle fémoral interne ou externe. L'état de la technique le plus proche est donné par le document US 4340978 A, qui définie le préambule des revendications dépendantes 1 et 8.

### ETAT ANTERIEUR DE LA TECHNIQUE

Dans la chirurgie orthopédique, il est connu de réaliser des prothèses uni-compartimentales de genou. Ces prothèses comprennent d'une manière générale un implant condylien métallique comprenant une surface interne d'ancrage dans un condyle d'un fémur, et une surface externe fonctionnelle, destinée à être en contact avec une partie d'usure. La surface fonctionnelle présente une courbure permettant l'articulation et les mouvements de flexion et d'extension du genou. Ces prothèses comprennent également un implant tibial comprenant une embase métallique d'ancrage avec le tibia recevant un insert fixe ou mobile en polyéthylène, communément appelé plateau tibial, et formant la partie d'usure sur laquelle est articulée l'implant condylien.

L'insert fixe est agencé entre les deux implants et est soumis à de fortes contraintes liées aux mouvements de flexion et d'extension du genou. En effet, pendant la flexion et l'extension du genou il existe un recul du fémur sur le tibia qui génère, du fait de la pression de l'implant condylien, une délamination de l'insert fixe, notamment une délamination suivant l'axe antéro-postérieur et une délamination suivant l'axe médio-latéral. En d'autres termes, lors des mouvements de flexion et d'extension du genou, le point de contact entre la surface fonctionnelle de l'implant condylien et l'insert fixe se déplace d'avant en arrière et de gauche à droite.

Aussi, pour permettre de réduire l'usure sur les inserts fixes, on utilise en implant condylien droit et symétrique pour assurer, tout au long de la flexion du genou, un point de contact sur l'insert qui se trouve dans un même plan sagittal, en évitant les mouvements suivant l'axe médio-latéral.

L'implant condylien présente donc une surface externe fonctionnelle comprenant plusieurs rayons de courbures successifs, et s'inscrit notamment dans une succession de sphères qui, lors de mouvement de flexion et d'extension du genou, roulent et glissent sur un plan.

L'implant condylien droit et symétrique permet donc de réduire l'usure et la délamination suivant l'axe médio-latéral.

Cependant, l'inconvénient réside dans le fait que les implants condyliens sont maintenant conçus pour s'adapter à la forme anatomique du patient, et comprennent des formes anatomiques ou biseautées, qui présentent le problème évoqué de délamination suivant les axes médio-latéral et antéro-postérieur.

Pour pallier à cet inconvénient de délamination de l'insert fixe, il est connu de mettre en oeuvre une prothèse uni-compartimentale comprenant un insert monté mobile sur l'embase d'ancrage de l'implant tibial et sur un axe antéro postérieur, de sorte que le recul du fémur entraine un recul postérieur de l'insert mobile.

De cette manière, cette mobilité permet de réduire significativement l'usure et la délamination de l'insert.

Cependant, la mise en place de cet insert mobile implique des coupes osseuses plus importantes pour permettre la mise en place, d'une embase d'ancrage tibial suffisamment résistante, comprenant notamment une épaisseur relativement importante de l'ordre de 3 à 4 mm, d'un insert épais d'environ 7 mm minimum, et d'un implant condylien de 6 mm, soit un total de 16 à 17 mm, contrairement aux prothèses à insert fixe plat qui permet une épaisseur d'embase de 1 à 2 mm, une épaisseur d'insert de 6 mm, et un implant condylien de 6 mm, soit un total de 13 à 14 mm, avec, par conséquent, un geste plus conservateur pour le capital osseux, sans risque de luxation. En effet, en cas d'instabilité de la prothèse à insert mobile, il peut être constaté des luxations d'insert par dé-coaptation.

### EXPOSE DE L'INVENTION

L'un des buts de l'invention est donc de remédier aux inconvénients précités en proposant un implant condylien pour prothèse de genou comme décrit dans la revendication 1, permettant de réduire l'usure dudit insert en évitant les mouvements de délaminations suivant l'axe médio-latéral, pour tout type de forme d'implant condylien, telle que symétrique, anatomique, ou biseautée par exemple.

A cet effet, il a été mis au point un implant condylien pour prothèse, de préférence uni-compartimentale, d'une articulation de genou, ledit implant comprenant une surface interne d'ancrage dans un condyle d'un fémur, et une surface externe fonctionnelle destinée à être en contact avec une partie d'usure de la prothèse, ladite surface externe fonctionnelle présentant une courbure permettant l'articulation et des mouvements de flexion et d'extension du genou.

Conformément à l'invention, la surface externe fonctionnelle présente uniquement deux courbures, dont :
- une première courbure s'inscrivant sur une surface de révolution d'un demi-cercle autour de son diamètre, et transversalement audit diamètre ;
- une deuxième courbure, dans la prolongation de la première courbure, s'inscrivant sur une surface de révolution d'un arc du demi-cercle autour d'une corde parallèle au diamètre, et transversalement à ladite corde.

On rappelle que par « corde », on entend un segment de droite joignant les extrémités de l'arc.

Ainsi, la surface externe fonctionnelle de l'implant condylien présente une courbure qui permet une cinématique et un comportement rhéologique identiques quelle que soit la forme dudit implant condylien, à savoir symétrique, anatomique ou biseautée, et sans mouvement suivant l'axe médio-latéral.

L'invention permet donc de réaliser des implants condyliens de forme anatomique pour assurer une couverture osseuse optimale sans dégrader les performances en termes d'usure. L'invention trouve une application aussi bien pour les prothèses uni-compartimentales que pour les prothèses bi-compartimentales ou tri-compartimentales de genou. De même, l'invention trouve une application aussi bien pour les prothèses à insert fixe que pour les prothèses à insert mobile.

De préférence, la première courbure est située sur une zone antérieure de la surface externe fonctionnelle.

De préférence, la deuxième courbure est située sur une zone postérieure de la surface externe fonctionnelle.

L'implant condylien selon l'invention peut présenter toute forme appropriée, l'essentielle réside qu'il présente les deux courbures précitées. Par exemple, la surface externe fonctionnelle de l'implant condylien peut s'étendre soit selon une forme anatomique adaptée à un patient, soit selon une forme droite et symétrique, ou bien soit selon une forme droite et biseautée.

L'ancrage de l'implant condylien sur le fémur peut se faire de toute manière appropriée, par exemple au moyen de plots d'ancrage agencés sur la surface interne dudit implant condylien.

### BREVE DESCRIPTION DES DESSINS

D'autres caractéristiques et avantages de l'invention ressortiront clairement de la description qui en est réalisée ci-après, à titre indicatif et nullement limitatif, en référence aux figures annexées dans lesquelles :
- la figure 1 est une vue schématique en perspective illustrant l'implant condylien selon l'invention présentant une surface fonctionnelle s'inscrivant dans une demi-sphère et une ellipsoïde de révolution ;
- la figure 2 est une vue schématique similaire à celle de la figure 1, l'implant condylien étant représenté vue de côté ;
- la figure 3 est une représentation schématique similaire à celle de la figure 1, l'implant condylien étant représenté de dessous et en perspective ;
- la figure 4 est une vue schématique similaire à celle de la figure 1, l'implant condylien étant représenté vue de dessous ;
- la figure 5 est vue schématique similaire à celle de la figure 1, l'implant condylien étant représenté vue de derrière ;
- la figure 6 est vue schématique similaire à celle de la figure 1, l'implant condylien étant représenté vue de face et s'étendant selon une forme anatomique ;
- la figure 7 est vue schématique similaire à celle de la figure 1, l'implant condylien étant représenté vue de face et s'étendant selon une forme biseautée ;
- la figure 8 est vue schématique similaire à celle de la figure 1, l'implant condylien étant représenté vue de face et s'étendant selon une forme symétrique.

### EXPOSE DETAILLE DE L'INVENTION

L'invention concerne implant condylien pour prothèse, de préférence uni-compartimentale de genou, par exemple pour une arthroplastie partielle du genou pour un patient présentant une usure localisée au niveau du condyle fémoral interne ou externe.

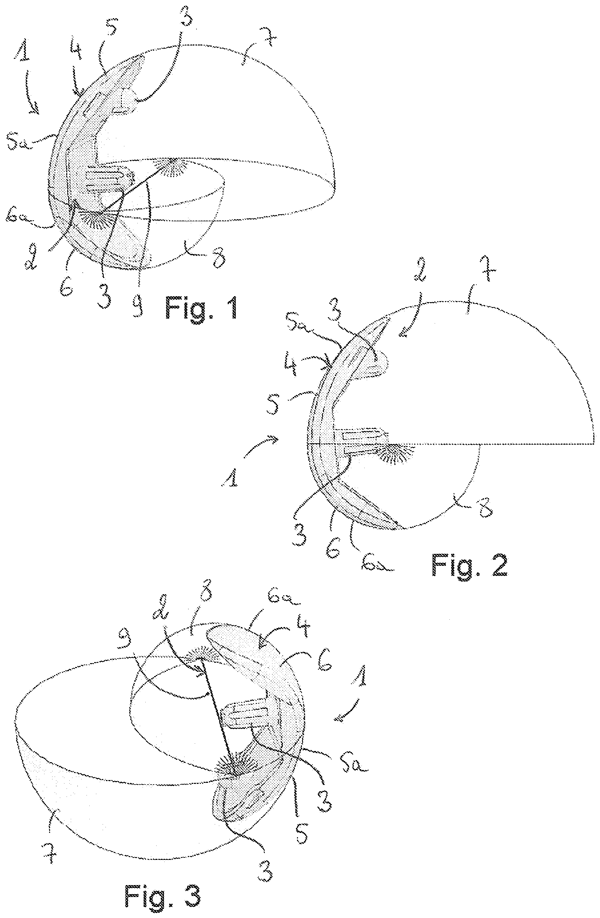

En référence aux figures 1 à 8, l'implant condylien (1) est métallique et comprend une surface interne (2), comprenant par exemple des plots d'ancrage (3), pour l'ancrage en tant que tel de l'implant condylien (1) dans un condyle d'un fémur, et une surface externe fonctionnelle (4), destinée à être en contact avec une partie d'usure.

La partie d'usure (non représentée), est constituée par un insert en polyéthylène monté, par exemple, d'une manière fixe ou mobile sur une embase d'ancrage dans un tibia.

La surface fonctionnelle (4) est destinée à être articulée sur cet insert fixe notamment pour les mouvements de flexion et d'extension du genou.

Dans les figures illustrées aux figures 1 à 6, la surface fonctionnelle (4) s'étend selon une forme anatomique et comprend une zone antérieure (5) et une zone postérieure (6) présentant des courbures différentes afin de réduire l'usure et éviter les mouvements de délamination de l'insert suivant l'axe médio-latéral lors des mouvements de flexion et d'extension du genou.

A cet effet, la zone antérieure (5) présente une première courbure (5a) s'inscrivant sur une surface de révolution (7) d'un demi-cercle autour de son diamètre. Ladite zone antérieure (5) s'inscrit sur la surface de révolution (7) transversalement au diamètre du demi-cercle.

La zone postérieure (6), quant à elle, présente une deuxième courbure (6a) s'inscrivant sur une surface de révolution (8) d'un arc du demi-cercle et autour d'une corde (9) parallèle au diamètre dudit demi-cercle. Ladite zone postérieure (6) s'inscrit transversalement à ladite corde (9) et dans la prolongation de la zone antérieure (5).

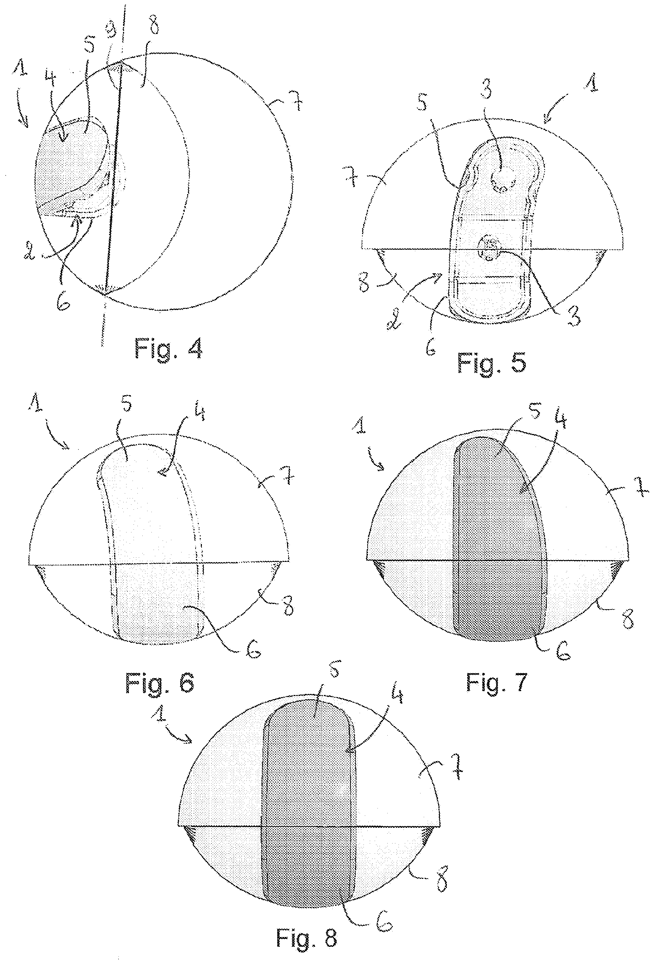

La configuration spécifique de la surface fonctionnelle (4) de l'implant condylien (1) selon l'invention permet une cinématique et un comportement rhéologique identiques quelle que soit la forme dudit implant condylien (1), à savoir biseautée (figure 7), anatomique ou symétrique (figure 8), et sans mouvement de délamination de l'insert suivant l'axe médio-latéral.

L'invention permet donc de réaliser des implants condyliens (1) de forme anatomique pour assurer une couverture osseuse optimale sans dégrader les performances en termes d'usure.

## Revendications

1. Implant condylien (1) pour prothèse uni-compartimentale d'une articulation de genou, ledit implant comprenant une surface interne (2) d'ancrage dans un condyle d'un fémur, et une surface externe fonctionnelle (4) destinée à être en contact avec une partie d'usure de la prothèse, ladite surface externe fonctionnelle (4) présentant une courbure permettant l'articulation et des mouvements de flexion et d'extension du genou, ***caractérisé* en ce que** la surface externe fonctionnelle (4) présente uniquement deux courbures, dont :
- une première courbure (5a) s'inscrivant sur une surface (7) obtenue par révolution d'un demi-cercle autour de son diamètre, ladite première courbure (5a) s'inscrivant transversalement audit diamètre ;
- une deuxième courbure (6a) s'inscrivant sur une surface (8) obtenue par révolution d'un arc du demi-cercle autour d'une corde (9) dudit demi-cercle, ladite corde (9) étant parallèle au diamètre du demi-cercle, et ladite deuxième courbure (6a) s'inscrivant dans la prolongation de la première courbure (5a) et transversalement à ladite corde (9).

2. Implant condylien (1) selon la revendication 1, ***caractérisé* en ce que** la première courbure (5a) est située sur une zone antérieure (5) de la surface externe fonctionnelle (4).

3. Implant condylien (1) selon l'une quelconque des revendications 1 à 2, ***caractérisé* en ce que** la deuxième courbure (6a) est située sur une zone postérieure (6) de la surface externe fonctionnelle (4).

4. Implant condylien (1) selon l'une quelconque des revendications 1 à 3, ***caractérisé* en ce qu'**il comprend une forme anatomique adaptée à un patient.

5. Implant condylien (1) selon l'une quelconque des revendications 1 à 3, ***caractérisé* en ce qu'**il comprend une forme droite et symétrique.

6. Implant condylien (1) selon l'une quelconque des revendications 1 à 3, ***caractérisé* en ce qu'**il comprend une forme droite et biseautée.

7. Implant condylien (1) selon l'une quelconque des revendications 1 à 6, ***caractérisé* en ce que** la surface interne (2) comprend des plots d'ancrage (3).

8. Prothèse uni-compartimentale d'une articulation de genou, comprenant un implant condylien et une embase d'ancrage dans un tibia, l'embase d'ancrage comprenant une partie d'usure fixe, et l'implant condylien comprenant une surface interne (2) d'ancrage dans un condyle d'un fémur, et une surface externe fonctionnelle (4) destinée à être en contact avec la partie d'usure fixe, ladite surface externe fonctionnelle (4) présentant une courbure permettant l'articulation et des mouvements de flexion et d'extension du genou, ***caractérisée* en ce que** la surface externe fonctionnelle (4) présente uniquement deux courbures, dont :
- une première courbure (5a) s'inscrivant sur une surface (7) obtenue par révolution d'un demi-cercle autour de son diamètre, ladite première courbure (5a) s'inscrivant transversalement audit diamètre ;
- une deuxième courbure (6a) s'inscrivant sur une surface (8) obtenue par révolution d'un arc du demi-cercle autour d'une corde (9) dudit demi-cercle, ladite corde (9) étant parallèle au diamètre du demi-cercle, et ladite deuxième courbure (6a) s'inscrivant dans la prolongation de la première courbure (5a) et transversalement à ladite corde (9).

## Patentansprüche

1. Kondylenimplantat (1) für Einkammer-Prothese eines Kniegelenks, wobei dieses Implantat eine Innenfläche (2) zur Verankerung in einer Femurkondyle und eine funktionelle Außenfläche (4) umfasst, die dazu bestimmt ist, Kontakt zu einem Verschleißteil der Prothese zu haben, diese funktionelle Außenfläche (4) weist eine Krümmung auf, die das Biegen und Beuge- und Streckbewegungen des Knies ermöglicht, ***dadurch gekennzeichnet, dass*** die funktionelle Außenfläche (4) nur zwei Krümmungen aufweist, darunter:
- eine erste Krümmung (5a) auf einer Fläche (7), die sich durch Drehung um einen Halbkreis um ihren Durchmesser ergibt, diese erste Krümmung (5a) liegt in Querrichtung zu diesem Durchmesser;
- eine zweite Krümmung (6a) auf einer Fläche (8), die sich durch Drehung eines Halbkreisbogens um eine Sehne (9) dieses Halbkreises ergibt, diese Sehne (9) liegt parallel zum Durchmesser des Halbkreises und diese zweite Krümmung (6a) liegt in Verlängerung der ersten Krümmung (5a) und in Querrichtung zu dieser Sehne (9).

2. Kondylenimplantat (1) gemäß Anspruch 1, ***dadurch gekennzeichnet, dass*** die erste Krümmung (5a) in einer vorderen Zone (5) der funktionellen Außenfläche (4) liegt.

3. Kondylenimplantat (1) nach irgendeinem der Ansprüche 1 bis 2, **dadurch gekennzeichnet, dass** die zweite Krümmung (6a) in einer hinteren Zone (6) der funktionellen Außenfläche (4) liegt.

4. Kondylenimplantat (1) nach irgendeinem der Ansprüche 1 bis 3, ***dadurch gekennzeichnet, dass*** es eine an einen Patienten angepasste anatomische Form enthält.

5. Kondylenimplantat (1) nach irgendeinem der Ansprüche 1 bis 3, ***dadurch gekennzeichnet, dass*** es eine gerade und symmetrische Form enthält.

6. Kondylenimplantat (1) nach irgendeinem der Ansprüche 1 bis 3, ***dadurch gekennzeichnet, dass*** es eine gerade und abgekantete Form enthält.

7. Kondylenimplantat (1) nach irgendeinem der Ansprüche 1 bis 6, ***dadurch gekennzeichnet, dass*** die Innenfläche (2) Verankerungspunkte (3) enthält.

8. Einkammer- Prothese eines Kniegelenkes, mit einem Kondylenimplantat und einer Platte zur Verankerung in einer Tibia, die Verankerungsplatte umfasst ein festes Verschleißteil und das Kondylenimplantat umfasst eine Innenfläche (2) zur Verankerung in einer Femurkondyle und eine funktionelle Außenfläche (4), die dazu bestimmt ist, Kontakt zu einem Verschleißteil der Prothese zu haben, wobei diese funktionelle Außenfläche (4) eine Krümmung aufweist, die das Biegen und Beugeund Streckbewegungen des Knies ermöglicht, ***dadurch gekennzeichnet, dass*** die funktionelle Außenfläche (4) nur zwei Krümmungen aufweist, darunter:
- eine erste Krümmung (5a), auf einer Fläche (7), die sich durch Drehung um einen Halbkreis um ihren Durchmesser ergibt, diese erste Krümmung (5a) liegt in Querrichtung zu diesem Durchmesser;
- eine zweite Krümmung (6a), auf einer Fläche (8), die sich durch Drehung eines Halbkreisbogens um eine Sehne (9) dieses Halbkreises ergibt, diese Sehne (9) ist parallel zum Durchmesser des Halbkreises und diese zweite Krümmung (6a) liegt in Verlängerung der ersten Krümmung (5a) und in Querrichtung zu dieser Sehne (9).

## Claims

1. A condylar implant (1) for a unicompartmental prosthesis for a knee joint, said implant having an inner surface (2) for anchoring in a condyle of a femur, and a functional outer surface (4) designed to be in contact with a wearing portion of the prosthesis, said functional outer surface (4) having a curvature enabling the knee to undergo articulation and movements in flexion and in extension, said condylar implant being **characterized in that** the functional outer surface (4) has only two curvatures, namely:
- a first curvature (5a) defined by a surface (7) generated by rotating a semi-circle about the diameter of said semi-circle, said first curvature (5a) being defined such that it extends transversely to said diameter; and
- a second curvature (6a) defined by a surface (8) generated by rotating an arc of the semi-circle about a chord (9) of said semi-circle, said chord (9) being parallel to the diameter of the semi-circle, and said second curvature (6a) being defined such that it extends the first curvature (5a) and extends transversely to said chord (9).

2. A condylar implant (1) according to claim 1, **characterized in that** the first curvature (5a) is situated in an anterior region (5) of the functional outer surface (4).

3. A condylar implant (1) according to claim 1 or claim 2, **characterized in that** the second curvature (6a) is situated in a posterior region (6) of the functional outer surface (4).

4. A condylar implant (1) according to any one of claims 1 to 3, **characterized in that** it has an anatomical shape adapted to a patient.

5. A condylar implant (1) according to any one of claims 1 to 3, **characterized in that** it has a shape that is straight and symmetrical.

6. A condylar implant (1) according to any one of claims 1 to 3, **characterized in that** it has a shape that is straight and beveled.

7. A condylar implant (1) according to any one of claims 1 to 6, **characterized in that** the inner surface (2) is provided with anchoring studs (3).

8. A unicompartmental prosthesis for a knee joint, said unicompartmental prosthesis comprising a condylar implant and an anchoring base for anchoring in a tibia, the anchoring base having a stationary wearing portion, and the condylar implant having an inner surface (2) for anchoring in a condyle of a femur, and a functional outer surface (4) designed to be in contact with the stationary wearing portion, said functional outer surface (4) having a curvature enabling the knee to undergo articulation and movements in flexion and in extension, said unicompartmental prosthesis being **characterized in that** the functional outer surface (4) has only two curvatures, namely:
- a first curvature (5a) defined by a surface (7) generated by rotating a semi-circle about the diameter of said semi-circle, said first curvature (5a) being defined such that it extends transversely to said diameter; and
- a second curvature (6a) defined by a surface (8) generated by rotating an arc of the semi-circle about a chord (9) of said semi-circle, said chord (9) being parallel to the diameter of the semi-circle, and said second curvature (6a) being defined such that it extends the first curvature (5a) and extends transversely to said chord (9).
